(19) Europäisches Patentamt / European Patent Office / Office européen des brevets

(11) **EP 2 489 432 B1**

(12) **EUROPÄISCHE PATENTSCHRIFT**

(45) Veröffentlichungstag und Bekanntmachung des Hinweises auf die Patenterteilung:
**23.08.2017  Patentblatt 2017/34**

(21) Anmeldenummer: **11191520.3**

(22) Anmeldetag: **01.12.2011**

(51) Int Cl.:
*B01D 11/04* (2006.01)  *C07C 67/62* (2006.01)
*C07C 227/40* (2006.01)  *C07C 229/08* (2006.01)
*B01J 39/04* (2017.01)  *B01J 39/16* (2017.01)
*C13B 20/14* (2011.01)  *C02F 1/26* (2006.01)
*C02F 101/34* (2006.01)  *C02F 1/42* (2006.01)

(54) **VERFAHREN ZUR ENTFERNUNG EINER ORGANISCHEN VERBINDUNG AUS EINER WÄSSRIGEN LÖSUNG MITTELS EINES FLÜSSIGEN KATIONENAUSTAUSCHERS SOWIE REAKTIONSMISCHUNG ZU DIESEM ZWECK**

PROCESS FOR THE REMOVAL OF AN ORGANIC COMPOUND FROM AN AQUEOUS SOLUTION WITH A LIQUID CATION EXCHANGER, FURTHER A REACTION MIXTURE FOR THIS PURPOSE

PROCÉDÉ POUR L'EXTRACTION D'UN COMPOSÉ ORGANIQUE D'UNE SOLUTION AQUEUSE À L'AIDE D'UN ÉCHANGEUR DE CATIONS LIQUIDE, AINSI QU'UN MÉLANGE REACTIONNEL DESTINÉ À CE BUT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.02.2011  EP 11154707**

(43) Veröffentlichungstag der Anmeldung:
**22.08.2012  Patentblatt 2012/34**

(73) Patentinhaber: **Evonik Degussa GmbH**
**45128 Essen (DE)**

(72) Erfinder:
• **Erhardt, Frank**
**33604 Bielefeld (DE)**
• **Pfennig, Andreas**
**52074 Aachen (DE)**
• **Przybylski, Marie-Dominique**
**52062 Aachen (DE)**
• **Haas, Thomas**
**48161 Münster (DE)**
• **Roos, Martin**
**45721 Haltern am See (DE)**
• **Demicoli, Daniel**
**45131 Essen (DE)**
• **Pötter, Markus**
**48149 Münster (DE)**
• **Schubert, Anja**
**46348 Raesfeld (DE)**
• **Pfeffer, Jan Christoph**
**45355 Essen (DE)**
• **Tacke, Thomas**
**63755 Alzenau (DE)**
• **Häger, Harald**
**59348 Lüdinghausen (DE)**

(56) Entgegenhaltungen:
**WO-A1-98/02411    DE-A1- 19 919 490**

• **TEMPLE D M, ET AL: "Liquid Ion-exchange Extraction of some Physiologically Active Amines", NATURE, Bd. 209, Nr. 5024, 12. Februar 1966 (1966-02-12), Seiten 714-715, XP002637507, ISSN: 0028-0836, DOI: 10.1038/209714a0**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Anmeldung betrifft ein Verfahren zum Entfernen einer eine oder mehrere positive Ladungen aufweisenden organischen Verbindung aus einer wässrigen Lösung. Ein grundlegendes Problem bei der von nachwachsenden Rohstoffen ausgehenden biotechnologischen Herstellung von Feinchemikalien, die herkömmlicherweise ausgehend von fossilen Brennstoffen synthetisiert werden, besteht darin, das einmal erhaltene Produkt, das typischerweise in einer großvolumigen wässrigen Phase vorliegt, in eine organische Phase zu überführen. Diese Überführung führt man einerseits durch, um ein fertiges Zwischen- oder Endprodukt zu konzentrieren und um ggf. die synthetische Verarbeitung in folgenden Reaktionsschritten in organischer Lösung zu ermöglichen, andererseits, um die Ausbeute der Reaktion in der wässrigen Phase durch das Entfernen des erwünschten Produktes zu verbessern oder den Ablauf der Reaktion in einem technisch sinnvollen Rahmen überhaupt erst zu ermöglichen. Die direkte thermische Aufkonzentrierung des häufig in niedrigen Konzentrationen vorliegenden Produktes aus der großvolumigen wässrigen Lösung ist in der Regel nicht sinnvoll.

Die Verteilung einer Verbindung in einem Zweiphasensystem umfassend eine wässrige, hydrophile Phase und eine organische, hydrophobe Phase, die sich nicht mischen, hängt maßgeblich von den physikochemischen Eigenschaften der jeweiligen Verbindung ab. Während Verbindungen mit einem hohen Anteil an oder ausschließlich bestehend aus unsubstituierten Kohlenwasserstoffen sich überwiegend in der hydrophoben Phase anreichern, liegen Verbindung mit einem hohen Anteil an polaren Gruppen wie heteroatomhaltigen Funktionalitäten und ganz besonders Verbindungen mit Ladungen überwiegend oder praktisch ausschließlich in der wässrigen Phase vor, was eine Überführung in eine organische Phase erschwert.

Die Verteilung einer Verbindung in dem genannten Zweiphasensystem nach Einstellung des Gleichgewichts wird häufig mit Hilfe von Verteilungskoeffizienten, beispielsweise nach der Nernst'schen Gleichung

$$\alpha = c_{\text{Phase 1}} / c_{\text{Phase 2,}}$$

beschrieben. Ein spezieller Verteilungskoeffizient ist $K_{ow}$, auch als P-Wert bezeichnet, der das Verteilungsgleichgewicht einer Verbindung zwischen einer Oktanol- und einer wässrigen Phase charakterisiert:

$$K_{ow} = P = c_{\text{Oktanol}} / C_{\text{Wasser}}$$

Ein Beispiel für eine industriell stark nachgefragte, positiv geladene organische Verbindung stellen 12-Aminolaurinsäure (ALS) und deren Derivate, insbesondere der Methylester (ALSME) dar. ALS ist ein wichtiges Ausgangsprodukt bei der Herstellung von Polymeren, beispielsweise zur Herstellung von Leitungssystemen und Nylon. Herkömmlich wird ALS ausgehend von fossilen Rohstoffen in einem Prozess mit niedriger Ausbeute über Laurinlactam hergestellt, das durch Trimerisierung von Butadien, anschließende Hydrierung unter Bildung von Cyclododecan, anschließende Oxidation zu Cyclododecanon, Umsetzung mit Hydroxylaurin und anschließender Beckmann-Umlagerung synthetisiert wird. Ein vielversprechender Weg zur biotechnologischen Herstellung von ALS bzw. ALSME ist in der DE10200710060705 beschrieben.

Der Stand der Technik lehrt die Gewinnung positiv geladener organischer Verbindungen durch Kontaktieren einer wässrigen Reaktionsmischung umfassend ein biologisches Agens mit einer organischen Phase umfassend ein organisches Lösungsmittel. So beschreibt beispielsweise DE10200710060705 die Gewinnung des Produktes ALSME durch Ausschütteln mit Essigsäureethylester aus einem wässrigen Reaktionsgemisch. Asano *et al.* (2008) offenbaren die Extraktion von ALS mit Toluol aus einer wässrigen Reaktionslösung umfassend ein ALS synthetisierendes Enzym.

WO 98/02411 beschreibt ein Verfahren zur Aufreinigung von Aminosäuren, wobei eine die Aminosäure umfassende Phase mit einer mit dieser Phase nicht mischbaren Phase extrahiert wird. Die Phase, in welche extrahiert wird, enthält dabei Amine und Fettsäuren.

Der vorliegenden Erfindung daher liegt die Aufgabe zugrunde, ein Verfahren zur Entfernung positive geladener organischer Verbindungen, besonders ω-Aminocarbonsäuren, mit wenigstens einer positiven Ladung aus einer wässrigen Reaktionsmischung zu entwickeln, wobei eine möglichst vorteilhafte Lage der Verteilungsgleichgewichtes zwischen Reaktionsmischung und einer als Extraktionsmittel verwendeten hydrophoben organischen Phase erwünscht ist, d. h. das Verteilungsgleichgewicht soll möglichst weit auf der Seite der hydrophoben organischen Phase liegen.

Eine weitere der Erfindung zugrunde liegende Aufgabe besteht darin, ein Verfahren zur Entfernung organischer Verbindungen mit wenigstens einer positiven Ladung, besonders ω-Aminocarbonsäuren, aus einer wässrigen Lösung umfassend ein biologisches Agens unter Verwendung einer hydrophoben organischen Phase als Extraktionsmittel zu entwickeln, bei dem das Verteilungsgleichgewicht möglichst weit auf der Seite der hydrophoben organischen Phase liegt.

Eine weitere der Erfindung zugrunde liegende Aufgabe besteht darin, ein Verfahren zur Entfernung organischer Verbindungen mit wenigstens einer positiven Ladung, besonders ω-Aminocarbonsäuren, aus einer wässrigen Lösung unter Verwendung einer hydrophoben organischen Lösung als Extraktionsmittel zu entwickeln, das das Wachstum von biotechnologisch relevanten Mikroorganismen, insbesondere *Escherichia coli,* möglichst wenig beeinträchtigt bzw. verlangsamt und/oder die Zahl teilungsfähiger und/oder lebensfähiger und/oder respiratorisch aktiver und/oder stoffwechsel- und synthetisch aktiver Zellen dabei möglichst wenig verringert.

Schließlich liegt der Erfindung die Aufgabe zugrunde, ein Verfahren zur Entfernung einer organischen Verbindung mit wenigstens einer positiven Ladung, besonders ω-Aminocarbonsäuren, aus einer wässrigen Lösung umfassend ein biologisches Agens unter Verwendung einer hydrophoben organischen Phase als Extraktionsmittel aufzufinden, bei dem die Gesamtheit der für die Ausbeute, den Gesamtumsatz und schnelle Durchführbarkeit eines zugrundeliegenden biotechnologischen Syntheseverfahrens entscheidenden Eigenschaften, insbesondere die Toxizität der organischen Phase gegenüber dem biologischen Agens und die Aufnahme der Verbindung in das organische Extraktionsmittel, mit Hinblick auf die Gesamtausbeute oder einen schnelleren Ablauf oder, im Falle eines kontinuierlichen Prozesses, eine möglichst lange Verwendbarkeit des biologischen Agens optimiert ist, besonders für den Fall, dass die organische Verbindung mit wenigstens einer positiven Ladung das Produkt oder ein Zwischenprodukt des Syntheseverfahrens darstellt, das unter Beteiligung einer katalytischen Aktivität des biologischen Agens synthetisiert wird.

Diese und weitere Aufgaben werden durch den Gegenstand der beigefügten unabhängigen Ansprüche gelöst, wobei sich Ausführungsformen aus den Unteransprüchen ergeben.

Die der Erfindung zu Grunde liegende Aufgabe wird in einem ersten Aspekt gelöst durch ein Verfahren zum Entfernen einer eine oder mehrere positive Ladungen aufweisenden organischen Verbindung aus einer wässrigen Lösung, umfassend die Schritte

a) Bereitstellen der die organische Verbindung enthaltenden wässrigen Lösung und einer hydrophoben organischen Lösung, die einen flüssigen Kationenaustauscher umfasst,
wobei der flüssige Kationenaustauscher hydrophob ist,
b) Kontaktieren der wässrigen Lösung und der organischen Lösung, und
c) Abtrennen der organischen Lösung von der wässrigen Lösung,

wobei es sich bei der organischen Verbindung um eine Verbindung der Formel I

$$NH_3^+\text{-A-COOR}^1 \qquad (I),$$

handelt, wobei $R^1$ Wasserstoff, Methyl, Ethyl oder eine negative Ladung ist und A eine unsubstituierte, geradekettige Alkylengruppe mit wenigstens drei, bevorzugt wenigstens acht Kohlenstoffatomen ist,
und wobei es sich bei dem flüssigen Kationenaustauscher um eine ungesättige Fettsäure handelt.

In einer ersten Ausführungsform des ersten Aspektes wird das Problem gelöst durch ein Verfahren nach einem der Ansprüche 1, wobei die Temperatur bei Schritt b) 28 bis 70, bevorzugt 30 bis 37 °C beträgt.

In einer zweiten Ausführungsform, die auch eine Ausführungsform der ersten Ausführungsform des ersten Aspektes ist, wird das Problem gelöst durch ein Verfahren nach einem der Ansprüche 1 bis 2, wobei der pH-Wert bei Schritt b) 6 bis 8, bevorzugt 6,2 bis 7,2, beträgt.

In einer dritten Ausführungsform, die auch eine Ausführungsform der ersten bis zweiten Ausführungsform des ersten Aspektes ist, wird das Problem gelöst durch ein Verfahren, wobei das Stoffmengenverhältnis von flüssigem Kationenaustauscher zu organischer Verbindung wenigstens 1 beträgt.

In einer dritten Ausführungsform, die auch eine Ausführungsform der ersten bis dritten Ausführungsform des ersten Aspektes ist, wird das Problem gelöst durch ein Verfahren, wobei das Volumenverhältnis von organischer Lösung zu wässriger Lösung 1:10 bis 10:1 beträgt.

In einer vierten Ausführungsform, die auch eine Ausführungsform der ersten bis dritten Ausführungsform des ersten Aspektes ist, wird das Problem gelöst durch ein Verfahren, wobei der flüssige Kationenaustauscher eine Fettsäure mit mehr als 12, bevorzugt mit 14 bis 22, noch bevorzugter 16 bis 18 Kohlenstoffatomen ist.

In einer fünften Ausführungsform, die auch eine Ausführungsform der ersten bis vierten Ausführungsform des ersten Aspektes ist, wird das Problem gelöst durch ein Verfahren, wobei der flüssige Kationenaustauscher Ölsäure oder Erukasäure, ist.

In einer sechsten Ausführungsform, die auch eine Ausführungsform der ersten bis fünften Ausführungsform des ersten Aspektes ist, wird das Problem gelöst durch ein Verfahren, wobei die wässrige Lösung weiterhin eine Zelle umfasst.

In einer siebten Ausführungsform, die auch eine Ausführungsform der ersten bis sechsten Ausführungsform des ersten Aspektes ist, wird das Problem gelöst durch ein Verfahren, wobei die Zelle, bevorzugt eine bakterielle Zelle ist und die Zelle noch bevorzugter eine rekombinante Alkanmonooxygenase, eine rekombinante Transaminase sowie bevorzugt darüber hinaus wenigstens ein Enzym aus der Gruppe aufweist, die eine Alkoholdehydrogenase, eine Alanindehydrogenase und das AlkL-Genprodukt umfasst.

In einer achten Ausführungsform, die auch eine Ausführungsform der ersten bis siebten Ausführungsform des ersten Aspektes ist, wird das Problem gelöst durch ein Verfahren, wobei die organische Lösung darüber hinaus mindestens ein organisches Lösungsmittel enthält, bevorzugt eine Fettsäure und/oder einen Fettsäureester.

In einer neunten Ausführungsform, die auch eine Ausführungsform der ersten bis achten Ausführungsform des ersten Aspektes ist, wird das Problem gelöst durch ein Verfahren nach Anspruch 12, wobei die organische Lösung als flüssigen Kationenaustauscher 20 bis 80 Vol.-%, bevorzugt 25 bis 75 Vol.-% Ölsäure, und als Lösungsmittel Laurinsäuremethylester umfasst und es sich bei der organischen Verbindung um 12-Aminolaurinsäuremethylester handelt und in der wässrigen Lösung eine bakterielle Zelle anwesend ist, die eine rekombinante Alkanmonooxygenase, eine rekombinante Transaminase sowie bevorzugt darüber hinaus wenigstens ein Enzym aus der Gruppe aufweist, die eine Alkoholdehydrogenase, eine Alanindehydrogenase und das AlkL-Genprodukt umfasst.

In einem zweiten Aspekt wird das der Erfindung zu Grunde liegende Problem gelöst durch eine Reaktionsmischung umfassend eine wässrige Lösung und eine hydrophobe organische Lösung,

wobei die hydrophobe organische Lösung eine ungesättigte Fettsäure als flüssigen Kationenaustauscher umfasst,

und wobei die wässrige Lösung eine Verbindung der Formel (I)

$$NH_3^+\text{-}A\text{-}COOR^1 \qquad (I),$$

enthält, wobei $R^1$ Wasserstoff, Methyl, Ethyl oder eine negative Ladung ist und A eine unsubstituierte, geradekettige Alkylengruppe mit wenigstens drei, bevorzugt wenigstens acht Kohlenstoffatomen ist.

In einer Ausführungsform des zweiten Aspektes wird das der Erfindung zu Grunde liegende Problem gelöst durch eine Reaktionsmischung gemäß dem ersten Aspekt, wobei die wässrige Lösung weiterhin eine Zelle umfasst, die eine rekombinante Alkanmonooxygenase, eine rekombinante Transaminase sowie bevorzugt darüber hinaus wenigstens ein Enzym aus der Gruppe aufweist, die eine Alkoholdehydrogenase, eine Alanindehydrogenase und das AlkL-Genprodukt umfasst.

Die Erfinder der vorliegenden Erfindung haben gefunden, dass die Effizienz der Entfernung einer organischen Verbindung mit einer oder mehreren positiven Ladungen aus einer wässrigen Lösung in eine hydrophobe organische Lösung überraschend gesteigert werden kann, wenn diese organische Lösung einen flüssigen Kationenaustauscher umfasst. Ohne an irgendeine Theorie gebunden sein zu wollen, vermuten die Erfinder der vorliegenden Erfindung, dass die negative Ladung bzw. die negativen Ladungen des flüssigen Kationenaustauscher mit der einen positiven Ladung oder den mehreren positiven Ladungen der organischen Verbindungen ionisch interagiert/interagieren und dass diese Interaktion zu einer Maskierung wenigstens einer positiven Ladung führt, welche die Löslichkeit in der organischen Phase erhöht.

In einer bevorzugten Ausführungsform bedeutet der Begriff "flüssiger Kationenaustauscher", wie hierin verwendet, eine in einem hydrophoben organischen Lösungsmittel lösliche Verbindung, die aufgrund einer oder mehrerer negativer permanenter Ladungen in der Lage ist, eine ionische Wechselwirkung mit wenigstens einem Kation auszubilden. Typischerweise umfasst ein flüssiger Kationenaustauscher wenigstens eine gesättigte oder ungesättigte Kohlenwasserstoffkette, die geradkettig oder verzweigt sein kann sowie eine negative geladene Gruppe, beispielsweise eine Carboxygruppe. Gemäß der vorliegenden Erfindung handelt es sich bei dem flüssigen Ionenaustauscher um eine ungesättigte Fettsäure, beispielsweise Ölsäure.

In einer bevorzugten Ausführungsform weist der flüssige Ionenaustauscher nicht nur eine negative Gesamtladung, sondern gar keine positive Ladung auf. In einer bevorzugten Ausführungsform ist unter dem Begriff "Gesamtladung" des Ionenaustauschers oder eines sonstigen Moleküls, wie hierin verwendet, die Summe der Ladungen aller kovalent mit dem Molekül verbundenen funktionellen Gruppen zu verstehen. Beispielsweise weist Laurinsäure bei pH 7 eine negative Ladung als Gesamtladung auf, unabhängig von der Anwesenheit weiterer Moleküle oder Gegenionen wie Kaliumionen, die in der wässrigen Lösung vorhanden sind.

In einer bevorzugten Ausführungsform der vorliegenden Erfindung wird unter dem Begriff "Kontaktieren", wie hierin verwendet, verstanden, dass zwei Phasen direkt und insbesondere ohne Zwischenschaltung einer physikalischen Barriere wie einer Membran einander gegenüber exponiert werden. Das Kontaktieren erfolgt im einfachsten Fall dadurch, dass die beiden Phasen ins gleiche Gefäß gegeben und in geeigneter Weise, beispielsweise durch Rühren, mit einander vermischt werden.

In einer bevorzugten Ausführungsform weist die organische Verbindung eine positive Gesamtladung auf. In einer weiteren bevorzugten Ausführungsform weist die organische Verbindung keine negativen Ladungen auf. In einer bevorzugten Ausführungsform handelt es sich bei der organischen Verbindung um eine ω-Aminocarbonsäure.

In einer bevorzugten Ausführungsform bedeutet der Begriff "weist eine Ladung auf", wie hierin verwendet, dass eine so bezeichnete Verbindung eine entsprechende Ladung in wässriger Lösung bei pH 0 bis 14, bevorzugte 2 bis 12, 2 bis 6, 8 bis 12, 3 bis 10, 6 bis 8, am bevorzugtesten bei pH 7 aufweist. In einer bevorzugten Ausführungsform handelt es sich um eine permanent vorliegende Ladung. In einer weiteren bevorzugten Ausführungsform bedeutet der Begriff "weist eine Ladung auf", wie hierin verwendet, dass die entsprechende funktionelle Gruppe oder Verbindung bei pH 7 überwiegend mit der entsprechenden Ladung vorliegt, d.h. zu wenigstens 50, bevorzugter 90 noch bevorzugter 99 %.

In einer bevorzugten Ausführungsform der Erfindung ist der Begriff "enthaltend" im Sinne von "umfassend", d.h. nicht abschließend zu verstehen. Eine Mischung enthaltend A kann in diesem Sinne neben A weitere Bestandteile aufweisen. Die Formulierung "eine oder mehrere Ladungen" bedeutet wenigstens eine Ladung der entsprechenden Natur. Gemäß der vorliegenden Erfindung wird unter dem Begriff "hydrophob", wie hierin verwendet, die Eigenschaft einer Flüssigkeit verstanden, in Anwesenheit von einer wässrigen Phase eine eigene, von der wässrigen Phase klar abgegrenzte flüssige Phase auszubilden. Bei letzterer kann es sich um eine zusammenhängende flüssige Phase oder um eine Emulsion handeln. Gemäß der vorliegenden Erfindung wird mit dem Begriff "hydrophob", wie hierin verwendet, die Eigenschaft einer Verbindung verstanden, sich im Wesentlichen nicht in Wasser zu lösen. Schließlich wird der Begriff wie hierin verwendet, derart verstanden, dass eine derartige bezeichnete Verbindung einen P-Wert (J. Sangster, Octanol-Water Partition Coefficients: Fundamentals and Physical Chemistry, Vol. 2 of Wiley Series in Solution Chemistry, John Wiley & Sons, Chichester, 1997) aufweist, dessen dekadischer Logarithmus größer 0, bevorzugter größer 0,5, noch bevorzugter größer 1 und am bevorzugtesten größer 2 ist. Gemäß der vorliegenden Erfindung weist der flüssige Ionenaustauscher keine oder nur eine mäßige toxische Wirkung auf biotechnologisch relevante Mikroorganismen auf. Unter dem Begriff "toxische Wirkung", wie hierin verwendet, wird in einer bevorzugten Ausführungsform der Erfindung die Eigenschaft einer Verbindung verstanden, bei Kontakt mit den entsprechenden Mikroorganismen deren Wachstumsgeschwindigkeit zu senken, deren Stoffwechselaktivität zu senken, deren Energieverbrauch zu erhöhen, deren optische Dichte oder Zahl wachstumsfähiger Zellen zu senken und/oder direkt zu deren Absterben und Lyse zu führen. In einer bevorzugten Ausführungsform wird wenigstens eine dieser Wirkungen bei einer toxischen Verbindung bereits in geringer Konzentration, vorzugsweise bei einer Konzentration von 1000, bevorzugter 100, noch bevorzugter 50 oder 25, am bevorzugtesten 5 mg/L, erreicht. Dem Fachmann sind zahlreiche routinemäßig anwendbare Verfahren bekannt, mittels derer die Toxizität untersucht werden kann. Hierzu zählen beispielsweise die Messung der Atmung entsprechender Mikroorganismen über $O_2$-Elektroden oder das vergleichende Ausplattieren von Mikroorganismenproben und das anschließende Zählen der koloniebildenden Einheiten (cfus). In einer bevorzugten Ausführungsform wird unter einer "mäßigen toxischen Wirkung" verstanden, dass in einer Wachstumsphase befindliche Mikroorganismen in Anwesenheit der Verbindung weiter wachsen und/oder stoffwechselaktiv sind, jedoch in geringerem Maße als bei einer Kontrolle, die unter gleichen Bedingungen in Abwesenheit der entsprechenden Verbindung inkubiert wird, und/oder eine verlängerte Lag-Phase aufweisen.

Das Kontaktieren von wässriger und organischer Lösung findet unter geeigneten Bedingungen und insbesondere über einen Zeitraum statt, der für einen ausreichenden Übertritt der organischen Verbindung aus der wässrigen Phase in die organische Phase ausreicht, idealerweise sogar für die Einstellung des entsprechenden Gleichgewichts. Diese Zeitdauer und Bedingungen kann der Fachmann im Rahmen routinemäßigen Experimentierens bestimmen.

In einer besonders bevorzugten Ausführungsform handelt es sich bei der eine oder mehrere positive Ladungen aufweisenden organischen Verbindung um eine endständig aminierte Fettsäure, besonders bevorzugt 12-Aminolaurinsäure oder einen Ester davon oder einen Gemisch beider Verbindungen. Der Fachmann wird anerkennen, dass ein Ester einer Fettsäure in Anwesenheit eines biologischen Systems umfassend Esteraseaktivitäten teilweise in Form der entsprechenden Säure vorliegen kann und beide Verbindungen in diesem Zusammenhang insofern als äquivalent zu betrachten sind. Dementsprechend umfassen Fettsäuren oder Fettsäurederivate in einer besonders bevorzugten Ausführungsform, wie hierin verwendet, auch die entsprechenden Ester, bevorzugt Methylester, und umgekehrt. Gemäß der vorliegenden Erfindung handelt es sich bei dem Begriff "Alkylengruppe", wie hierin verwendet, um eine Gruppe der Formel - $(CH_2)_n$ -, d.h. um ein Alkan mit zwei offen gelassenen, bevorzugt endständigen Substituenten. Bei den zwei Substituenten kann es sich z.B. um eine Amingruppe und eine Carboxygruppe handeln. Gemäß der vorliegenden Erfindung beträgt n wenigstens 3, noch bevorzugter wenigstens 6, noch bevorzugter 11. Bei einer "substituierten Alkylenkette" ist wenigstens ein Wasserstoffatom durch einen anderen Substituenten als ein Wasserstoffatom oder einen Alkylrest, bevorzugt ein anderes Atom als ein Wasserstoffatom, ersetzt. Gemäß der vorliegenden Erfindung bedeutet der Begriff "unsubstituierte Alkylengruppe", wie hierein verwendet, hingegen eine Kohlenwasserstoffkette der Formel - $(CH_2)_n$ - ohne einen derartigen Substituenten

Die Temperatur bei Schritt b) hängt nicht nur von den Eigenschaften des flüssigen Kationenaustauschers ab, sondern, insbesondere für den Fall, dass das Kontaktieren der wässrigen und der organischen Lösung bei ablaufender Reaktion in der wässrigen Phase stattfindet, auch von den Temperaturerfordernissen etwaiger, in der wässrigen Phase stattfindenden Reaktionen. Insbesondere für den Fall, dass ein biologisches Agens wie eine lebende Zelle in der wässrigen Phase katalytisch aktiv ist, muss die Temperatur für die Erhaltung dieser Aktivität geeignet sein. In einer bevorzugten Ausführungsform beträgt die Temperatur bei Schritt b) 0 bis 100 °C, bevorzugter 20 bis 80 °C, 28 bis 70 °C, 30 bis 37 °C, 35 bis 40 °C.

Auch der pH-Wert bei Schritt b) muss den Erfordernissen etwaiger gleichzeitig ablaufender Reaktionen, der Stabilität von Edukten, Produkten, Zwischenprodukten oder Agenzien Rechnung tragen. In einer bevorzugten Ausführungsform beträgt der pH-Wert 3 bis 8, bevorzugter 6 bis 8, noch bevorzugter 6,2 bis 7,2.

Um die organische Verbindung aus der wässrigen Phase möglichst vollständig in die organische zu überführen, ist eine ausreichende Menge des flüssigen Kationenaustauschers erforderlich. In einer bevorzugten Ausführungsform der vorliegenden Erfindung beträgt das Stoffmengenverhältnis von flüssigem Kationenaustauscher und organischer Verbindung bei wenigstens einem Schritt, bei einem kontinuierlichen Prozess über den gesamten Ablauf der Reaktion summiert, wenigstens 1, d.h. pro Molekül der organischen Verbindung wird wenigstens ein Molekül flüssiger Kationenaustauscher eingesetzt. In einer noch bevorzugteren Ausführungsform ist das Verhältnis größer als 2, 3, 5, 10, 15, oder 20, bevorzugt 1,5 bis 3.

Das Volumenverhältnis der organischen Lösung zur wässrigen Lösung ist, zusammen mit dem Stoffmengenverhältnis Kationenaustauscher/organische Verbindung, für ein effizientes Verfahren bedeutend. In einer besonderen Ausführungsform beträgt es 100:1 bis 1:100, bevorzugter 20:1 bis 1:20, noch bevorzugter 10:1 bis 1:10, 4:1 bis 1:4, 3:1 bis 1:3 oder am bevorzugtesten 1:2 bis 2:1.

Gemäß der vorliegenden Erfindung wird eine ungesättigte Fettsäure als flüssiger Kationenaustauscher eingesetzt. Gemäß der vorliegenden Erfindung wird unter dem Begriff "Fettsäure", wie hierin verwendet, eine Carbonsäure, bevorzugt Alkansäure, mit wenigstens 6, bevorzugt 8, noch bevorzugter 10, am bevorzugtesten 12 Kohlenstoffatomen verstanden. In einer bevorzugten Ausführungsform handelt es sich um geradkettige Fettsäuren, in einer weiteren Ausführungsform um verzweigte. In einer weiteren bevorzugten Ausführungsform handelt es sich um eine geradkettige Fettsäure mit wenigstens 12 Kohlenstoffatomen umfassend eine Doppelbindung, bevorzugt an Position 9. In einer weiteren bevorzugten Ausführungsform handelt es sich um eine einfach ungesättigte Fettsäure, bei der sich die Doppelbindung an der Position 9 und/oder 11 befindet. In einer weiteren bevorzugten Ausführungsform handelt es sich bei dem flüssigen Kationenaustauscher um eine ungesättigte Fettsäure ausgewählt aus der Gruppe umfassend Ölsäure, Palmitoleinsäure und Gadoleinsäure und Icosensäure. In der bevorzugtesten Ausführungsform handelt es sich um Ölsäure. In einer besonders bevorzugten Ausführungsform handelt es sich um eine Fettsäure mit 6, 7, 8 9, 10,11, 12, 13, 14, 15, 16, 17, 18, 19, 20, 21, 22, 23, 24, 25, 26, 27, 28, 29, 30 Kohlenstoffatomen, bevorzugt mehr als 12, noch bevorzugter mehr als 14 Kohlenstoffatomen, noch bevorzugter mit 14 bis 28, 14 bis 22, am bevorzugtesten 16 bis 18 Kohlenstoffatomen.

In einer weiteren bevorzugten Ausführungsform wird als flüssiger Ionenaustauscher ein Gemisch verschiedener Fettsäuren eingesetzt, wie es beispielsweise in Form von Sojaöl oder Kugeldistelöl vorliegt. Dies umfasst bei Bedarf eine vorherige Hydrolyse, falls die Fettsäuren als Ester vorliegen.

In einer besonders bevorzugten Ausführungsform der vorliegenden Erfindung wird eine Kombination von zwei flüssigen Kationenaustauschern, wenigstens einer von ihnen eine Fettsäure, verwendet. Ein besonderer Vorteil der vorliegenden Erfindung liegt in der Kompatibilität des erfindungsgemäßen Verfahrens mit biotechnologischen Verfahren und dabei verwendeten biologischen Agenzien. In einer besonderen Ausführungsform der vorliegenden Erfindung wird unter dem Begriff "biologisches Agens mit katalytischer Aktivität", wie hierin verwendet, ein durch eine Zelle synthetisierter Biokatalysator in sämtlichen Aufreinigungsstufen, von der gesamten Zelle bis zum isolierten Molekül, verstanden. In einer bevorzugten Ausführungsform handelt es sich um eine Zelle exprimierend Enzyme mit katalytischer Aktivität. Bei der Zelle kann es sich um einen Prokaryonten, einschließlich Archeen, oder um einen Eukaryonten handeln, bevorzugt aus der Gruppe umfassend *Pseudomonas, Corynebacterium* und *E. coli.* In einer noch bevorzugteren Ausführungsform handelt es sich bei dem Agens um eine bakterielle Zelle, noch bevorzugter um eine Gram-negative bakterielle Zelle, am bevorzugtesten um *E. coli.* In einer weiteren bevorzugten Ausführungsform handelt es sich um eine eukaryontische Zelle, bevorzugter um eine Pilzzelle, noch bevorzugter um eine Hefezelle, am bevorzugtesten um *Saccharomyces* oder *Candida, Pichia, insbesondere Candida tropicalis.* Der Begriff "Zelle" wird, in einer besonderen Ausführungsform, in dieser Anmeldung gleichbedeutend und austauschbar mit dem Begriff "Mikroorganismus" verwendet. Weiterhin kann es sich bei der Zelle um eine isolierte Zelle oder eine Mischung von Kulturen handeln.

Die als biologisches Agens verwendete Zelle kann lebensfähig sein, oder es kann sich um eine Präparation davon, beispielsweise um eine Membranfraktion oder cytosolische Fraktion oder ein Rohextrakt der Zelle, handeln.

Wenn es sich bei dem biologischen Agens um ein isoliertes Molekül in verschiedenen Reinigungsstufen handelt, so kann dies sämtliche katalytische aktive, von einer Zelle hergestellte Moleküle handeln. In einer besonders bevorzugten Ausführungsform handelt es sich um ein Molekül aus der Gruppe umfassend Peptide, Polypeptide, Kohlenhydrate, Nukleinsäuren oder Mischformen davon. In einer noch bevorzugteren Ausführungsform handelt es sich um ein katalytisch wirksames Polypeptid. In einer weiteren bevorzugten Ausführungsform handelt es sich um ein immobilisiertes Molekül.

Die für synthetische biotechnologische Verfahren erforderlichen katalytischen Funktionen sind vielfältig. In einer bevorzugten Ausführungsform handelt es sich beim Begriff "katalytische Aktivität", wie hierin verwendet, um eine synthetische Aktivität, d.h. die Katalyse chemischer Reaktionen umfassend die Bildung wenigstens einer neuen kovalenten Bindung. In einer weiteren bevorzugten Ausführungsform handelt es sich um eine Transportaktivität, d.h. die Fähigkeit eine Moleküle, den Transport eines anderen Moleküls von einem Kompartiment in ein anderes zu bewerkstelligen, z.B. die Aufnahme eines Stoffes aus dem wässrigen Medium über eine Zellmembran ins Innere der Zelle.

In einer besonders bevorzugten Ausführungsform handelt es sich bei dem biologischen Agens um eine lebende Zelle, die in Anwesenheit des flüssigen Kationenaustauschers zur Katalyse verwendet wird, bevorzugt, um eine organische Verbindung mit einer oder mehreren positiven Ladungen zu synthetisieren, die anschließend oder gleichzeitig mittels des flüssigen Kationenaustauschers in eine hydrophobe organische Phase entfernt wird.

In einer besonders bevorzugten Ausführungsform wirkt sich die Anwesenheit der organischen Verbindung nachteilig auf die katalytische Aktivität aus. In einer Ausführungsform kann dies die Menge an vorhandener Aktivität, ausdrückbar im Sinne eines niedrigeren $k_{cat}$ eines Enzyms, verringert. In einer weiteren Ausführungsform kann die Affinität des die katalytische Aktivität aufweisenden Agens im Sinne eines erhöhten $K_M$ eines Enzyms betroffen sein. In einer weiteren Ausführungsform kann die Spezifität der katalytischen Aktivität verändert sein, beispielsweise in der Weise, dass es bevorzugt ein anderes als das erwünschte Substratmolekül umsetzt oder bevorzugt umsetzt. In einer weiteren Ausführungsform weist die organische Verbindung eine toxische Wirkung auf eine Zelle als biologisches Agens auf.

In einer weiteren Ausführungsform handelt es sich bei der organischen Verbindung um einer organische Verbindung, die die Verfügbarkeit eines essentiellen Co-Substrates oder Co-Enzymes verringert. Dies kann z.B. der Fall sein, wenn die organische Verbindung eine entsprechende Regenerationsreaktion inhibiert.

Neben dem flüssigen Kationenaustauscher kann die hydrophobe organische Phase weiterhin ein hydrophobes Lösungsmittel enthalten. Dies kann dazu dienen, die Aufnahmekapazität eines flüssigen Kationenaustauschers in der hydrophoben Phase zu erhöhen und unerwünschtes Verhalten, beispielsweise Ausflocken, zu verhindern. In einer bevorzugten Ausführungsform handelt es sich bei dem Lösungsmittel um ein Edukt einer in der wässrigen Lösung ablaufenden Reaktion, am stärksten bevorzugt das Substrat einer in der wässrigen Lösung ablaufenden enzymkatalysierten Reaktion. In einer bevorzugten Ausführungsform handelt es sich um einen Fettsäureester. In einer besonders bevorzugten Ausführungsform handelt es sich bei dem Lösungsmittel um einen Fettsäureester, bevorzugt -methylester, einer Fettsäure, die als flüssiger Kationenaustauscher dient.

Der Anteil des Lösungsmittels, sofern vorhanden, an der hydrophoben organischen Phase beträgt in einer bevorzugten Ausführungsform 1 bis 99 Volumenprozent (Vol.-%). In einer bevorzugten Ausführungsform beträgt der Anteil des Lösungsmittels 10 bis 90, noch bevorzugter 20 bis 80, am bevorzugtesten 25 bis 75 Vol.-%.

In einer bevorzugtesten Ausführungsform des Verfahrens handelt es sich bei der organischen Verbindung um 12-Aminolaurinsäure und/oder 12-Aminolaurinsäuremethylester, das bzw. der in der wässrigen Phase von einem rekombinanten *E. coli*-Stamm durch schrittweise Oxidation des endständigen Kohlenstoffatoms von Laurinsäuremethylester hergestellt wird, wie es in der DE10200710060705 offenbart ist, und die hydrophobe Phase umfasst 25 bis 75 % Ölsäure als flüssigen Kationenaustauscher gelöst in Laurinsäuremethylester als Substrat der Reaktion.

Die Lehre der vorliegenden Erfindung kann nicht nur unter Verwendung der exakten Aminosäure- oder Nukleinsäuresequenzen der hierin beschriebenen biologischen Makromoleküle ausgeführt werden, sondern auch unter Verwendung von Varianten derartiger Makromoleküle, die durch Deletion, Addition oder Substitution einer oder mehr als einer Aminosäuren oder Nukleinsäuren erhalten werden können. In einer bevorzugten Ausführungsform bedeutet der Begriff "Variante" einer Nukleinsäuresequenz oder Aminosäuresequenz, im Folgenden gleichbedeutend und austauschbar mit dem Begriff "Homologon" gebraucht, wie hierin verwendet, eine andere Nukleinsäure- oder Aminosäuresequenz, die mit Hinblick auf die entsprechende ursprüngliche Wildtyp-Nukleinsäure- oder -aminosäuresequenz eine Homologie, hier gleichbedeutend mit Identität verwendet, von 70, 75, 80, 85, 90, 92, 94, 96, 98, 99 % oder mehr Prozent aufweist, wobei bevorzugt andere als die das katalytisch aktive Zentrum ausbildende Aminosäuren oder für die Struktur oder Faltung essentielle Aminosäuren deletiert oder substituiert sind oder letztere lediglich konservativ substituiert sind, beispielsweise ein Glutamat statt einem Aspartat oder ein Leucin statt einem Valin. Der Stand der Technik beschreibt Algorithmen, die verwendet werden können, um das Ausmaß von Homologie von zwei Sequenzen zu berechnen, z. B. Arthur Lesk (2008), Introduction to bioinformatics, 3rd edition. In einer weiteren bevorzugteren Ausführungsform der vorliegenden Erfindung weist die Variante einer Aminosäure- oder Nukleinsäuresequenz, bevorzugt zusätzlich zur oben genannten Sequenzhomologie, im Wesentlichen die gleiche enzymatisch Aktivität des Wildtypmoleküls bzw. des ursprünglichen Moleküls auf. Zum Beispiel weist eine Variante eines als Protease enzymatisch aktiven Polypeptids die gleiche oder im Wesentlichen die gleiche proteolytische Aktivität wie das Polypeptidenzym auf, d.h. die Fähigkeit, die Hydrolyse einer Peptidbindung zu katalysieren. In einer besonderen Ausführungsform bedeutet der Begriff "im Wesentlichen die gleiche enzymatische Aktivität" eine Aktivität mit Hinblick auf die Substrate des Wildtyp-Polypeptids, die deutlich über der Hintergrundaktivität liegt oder/und sich um weniger als 3, bevorzugter 2, noch bevorzugter eine Größenordnung von den $K_M$- und/oder $k_{cat}$- Werten unterscheidet, die das Wildtyppolypeptid mit Hinblick auf die gleichen Substrate aufweist. In einer weiteren bevorzugten Ausführungsform umfasst der Begriff "Variante" einer Nukleinsäure- oder Aminosäuresequenz wenigstens einen aktiven Teil/oder Fragment der Nukleinsäure- bzw. Aminosäuresequenz. In einer weiteren bevorzugten Ausführungsform bedeutet der Begriff "aktiver Teil", wie hierin verwendet, eine Aminosäuresequenz oder eine Nukleinsäuresequenz, die eine geringere als die volle Länge der Aminosäuresequenz aufweist bzw. für eine geringere als die volle Länge der Aminosäuresequenz kodiert, wobei die Aminosäuresequenz oder die kodierte Aminosäuresequenz mit geringerer Länge als der Wildtyp-Aminosäuresequenz im Wesentlichen die gleiche enzymatische Aktivität wie das Wildtyppolypeptid oder eine Variante davon aufweist, beispielsweise als Alkoholdehydrogenase, Monooxygenase oder Transaminase. In einer besonderen Ausführungsform umfasst der Begriff "Variante" einer Nukleinsäure eine Nukleinsäure, deren komplementärer Strang, bevorzugt unter stringenten Bedingungen, an die Wildtyp-Nukleinsäure bindet. Die Stringenz der Hybridisierungsreaktion ist für den Fachmann leicht bestimmbar und hängt im Allgemeinen von der Länge der Sonde, den Temperaturen beim Waschen und der Salzkonzentration ab. Im Allgemeinen benötigen längere Sonden höhere Temperaturen zum Hybridisieren, wohingegen kürzere Proben mit geringen Temperaturen auskommen. Ob Hybridisierung stattfindet, hängt im Allgemeinen von der Fähigkeit der denaturierten DNA ab, an komplementäre Stränge zu annellieren, die in ihrer Umgebung vorhanden sind, und zwar unterhalb der Schmelztemperatur. Die Stringenz von Hybridisierungsreaktion und entsprechende Bedingungen sind ausführlicher in Ausubel *et al. 1995* beschrieben. In einer bevorzugten Ausführungsform umfasst der Begriff "Variante" einer Nukleinsäure, wie hierin verwendet, eine beliebige Nukleinsäuresequenz, die für die gleiche Aminosäuresequenz wie die ursprüngliche Nukleinsäure oder eine Variante dieser Aminosäuresequenz im Rahmen der Degeneriertheit des genetischen Codes kodiert.

Geeignete Polypeptide, die zur Herstellung von organischen Verbindungen der Formel (I) verwendet werden können, insbesondere Alkanmonooxygenasen, AlkL, Transaminasen, Aldehyddehydrogenasen und Alanindehydrogenasen sind im Stand der Technik beschrieben, beispielsweise in der DE10200710060705, der EP11004029 oder in der PCT/EP2011/053834.

In der bevorzugtesten Ausführungsform handelt es sich bei der Alkanmonooxygenase um eine Alkanmonooxygenase des AlkB-Typs. AlkB stellt eine Oxidoreduktase aus dem AlkBGT-System aus *Pseudomonas putida* dar, die für ihre Hydroxylase-Aktivität bekannt ist. Diese ist von zwei weiteren Polypeptiden, AlkG und AlkT abhängig. AlkT wird als FAD-abhängige Rubredoxin-Reduktase charakterisiert, die Elektronen aus NADH an AlkG weitergibt. AlkG ist ein Rubredoxin, ein eisenhaltiges Redoxprotein, das als direkter Elektronendonor für AlkB fungiert. In einer bevorzugten Ausführungsform wird unter dem Begriff "Alkanmonooxygenase des alkB-Typs", wie hierin verwendet, eine membranständige Alkanmonooxidase verstanden. In einer weiteren bevorzugten Ausführungsform wird unter dem selben Begriff "Alkanmonoxygenase des alkB-Typs" ein Polypeptid mit einer Sequenzhomologie von zunehmend bevorzugt wenigstens 75, 80, 85, 90, 92, 94, 96, 98 oder 99 % zur Sequenz des AlkB von *Pseudomonas putida* Gpol (Datenbankcode: CAB54050.1) verstanden. In einer weiteren bevorzugten Ausführungsform wird unter dem Begriff eine Cytochrom-unabhängige Monooxygenase verstanden. In einer weiteren bevorzugten Ausführungsform wird unter dem Begriff "Alkanmonooxygenase des alkB-Typs" 5 eine cytochromunabhängige Monooxygenase verstanden, die wenigstens ein Rubredoxin oder Homologon als Elektronendonor verwendet. In einer besonders bevorzugten Ausführungsform wird unter dem Begriff eine membranständige, Cytochrom-unabhänige Alkanmonooxygenase mit zunehmend bevorzugt wenigstens 60, 70, 80, 80, 85, 90, 92, 94, 96, 98 oder 99 % zur Sequenz des AlkB von *Pseudomonas putida* Gpo 1 verstanden, die als Elektronendonor wenigstens AlkG (CAB54052.1), bevorzugt aber die Kombination von AlkG mit der Reduktase AlkT (CAB54063.1) benötigt, wobei es sich bei alkG und/oder alkT auch um ein Homologon des jeweiligen Polypeptides handeln kann. Der Begriff "Sequenz", wie hierin verwendet, kann sich auf die Aminosäuresequenz eines Polypeptides und/oder die dafür codierende Nukleinsäuresequenz beziehen. In einer weiteren bevorzugten Ausführungsform handelt es sich bei einer "Alkanmonooxygenase des alkB-Typs", wie hierin verwendet, um eine Cytochrom-unabhängige Oxidoreduktase, d.h. eine Oxidoreduktase, die nicht Cytochrom als Cofaktor umfasst.

Die vorliegende Erfindung wird weiterhin durch die folgenden Figuren und nicht beschränkenden Beispiele veranschaulicht, denen weitere Merkmale, Ausführungsformen, Aspekte und Vorteile der vorliegenden Erfindung entnommen werden können.

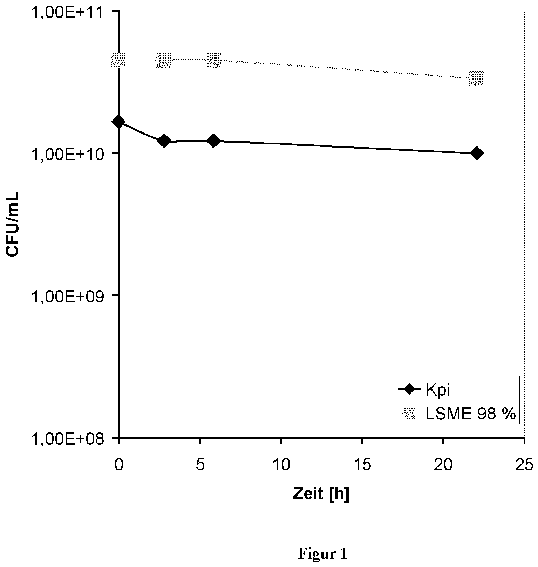

**Fig. 1** zeigt ein Kontrollexperiment zur Bestätigung, dass LSME nicht toxisch wirkt, untersucht mit einem *E. coli* W3110-Stamm und im Vergleich zu Kaliumphosphatpuffer (Kpi) als Negativkontrolle.

**Fig. 2** zeigt die Lebensfähigkeit des Stammes *E. coli* W3110 in Form von der Zahl der cfus, die der Stamm in Abwesenheit eines flüssigen Kationenaustauschers und in Anwesenheit verschiedener flüssiger Kationenaustauscher nach 0 h, 4 h und 24 h ausbilden kann.

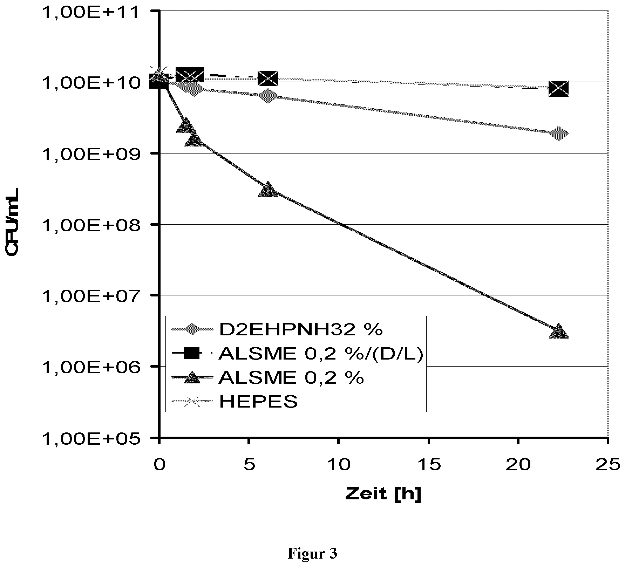

**Fig. 3** zeigt den Effekt der Verwendung eines flüssigen Kationenaustauschers auf die Toxizität anhand der Veränderung der Lebendzellzahl von einem *E. coli*-W3110-Stamm in Gegenwart von ALSME 0,2 %, mit Ammoniak eingestelltem DEHPA ("D2EHPNH3 2%") bzw. einer DEHPA/LSME Mischung (2 %/98 %) ("D/L") in Anwesenheit von ALSME 0,2%.

**Fig. 4** zeigt den Effekt verschiedener flüssiger Kationenaustauscher auf die OTR von Aminolaurinsäuremethylester produzierenden *E. coli*-Stammes. Das Experiment wurde wie in Beispiel 4 beschrieben durchgeführt.

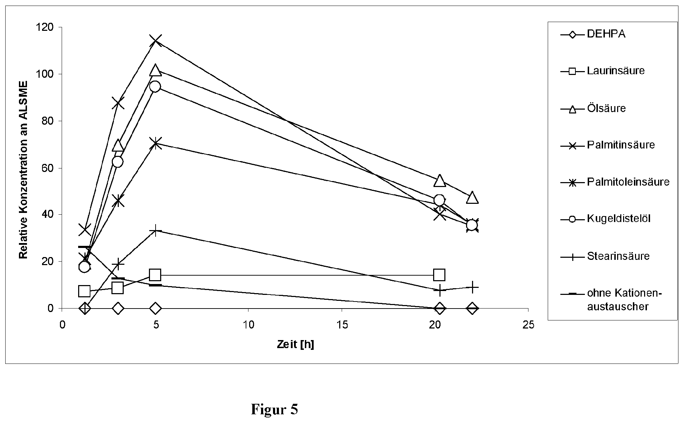

**Fig. 5** zeigt den Einfluss verschiedener flüssiger Kationenaustauscher auf die Ausbeute an Aminolaurinsäuremethylester, die ein *E. coli*-Stamm mit geeigneter genetischer Modifikation herstellt. Das Experiment wurde wie in Beispiel 4 beschrieben durchgeführt.

**Beispiel 1: Untersuchung zur Toxizität des Lösungsmittels LSME, das in Zusammensetzungen mit flüssigen Kationenaustauschern verwendet wird.**

Mit diesem Versuch wurde die relativ geringe Toxizität von LSME mit Hinblick auf biotechnologisch relevante Mikroorganismen gezeigt, die LSME zu einem geeigneten organischen Lösungsmittel für das erfindungsgemäße Verfahren macht.

Bevor die Bestimmung der CFU durchgeführt werden konnte, wurde eine Platte LB (10 g/L Pepton aus Casein, 5 g/L Hefeextrakt, 10 g/L NaCl) mit *E. coli* BW3110 ausgestrichen und für 24 h inkubiert. Am Abend des darauf folgenden Tages wurde eine Vorkultur von dieser zuvor ausgestrichenen Platte angeimpft. Diese Vorkultur hatte ein Volumen von 50 mL LB-Medium, und wurde über Nacht für ca. 16 h inkubiert. Am folgenden Tag wurde die Vorkultur mit einer $OD_{600}$ von 0,2 in 200 mL M9-Medium (Na2HPO4 6,79 g/L; KH2PO4 3,0 g/L; NaCl 0,5 g/L; NH4Cl 1g/L; 1 mL/L Spurenelementelösung, pH 7,4. Spurenelementlösung: HCl 37 % (=455,8 g/L) 36,50 g/L; MnCl2*7H2O 1,91 g/L; ZnSO4*7H2O 1,87 g/L; Na-EDTA*2H2O (Titriplex III) 0,84 g/L; H3BO3 0,30 g/L; Na2MoO4*2H2O 0,25 g/L; CaCl2*2H2O 4,70 g/L; FeSO4*7H2O 17,80 g/L; CuCl2*2H2O 0,15 g/L) mit 3 % Glucose (w/v) überimpft und für ca. 20 h inkubiert. Nach der Inkubation der Hauptkultur wurden die Zellen geerntet, bei 5258 g und 4 °C für 10 min zentrifugiert und mit einer $OD_{600}$ von 30 in 10 mL 50mM $Kp_i$-Puffer bei pH 7,4 (oder 25mM HEPES-Puffer pH 7,4, wenn CFU-Bestimmungen mit ALSME durchgeführt wurden) resuspendiert. Beide verwendeten Pufferlösungen enthielten 5 % Glucose (w/v). Anschließend wurde die Bakteriensuspension in die Schüttelkolben überführt und mit den jeweiligen Substanzlösungen versetzt. Nach erfolgter Durchmischung durch Schwenken des Kolbens wurden 100 μL der Suspension heraus pipettiert und in 900 μL vorgelegte sterile Saline gefüllt. Dies entsprach der Probenahme zum Zeitpunkt $t_0$. Es folgte die Inkubation der Ansätze bei 250 Upm und 30 °C. Die CFUs wurden über einen Zeitraum von 22 h ermittelt. Die Probenahmen fanden zunächst zu den Zeitpunkten $t_0$, $t_3$, $t_6$ und $t_{22}$ statt. Bei manchen Ansätzen wurde ein weiterer Probenahmezeitpunkt $t_{1,5}$ hinzugefügt und darüber hinaus eine weitere zusätzliche Verdünnungsreihe ausplattiert, um Abweichungen zu minimieren.

Die $OD_{600}$ lag bei 60. Die Zellen wurden in 10 mL $Kp_i$-Puffer resuspendiert und anschließend im Kolben mit 5 mL LSME 98 % (w/w) vermischt. Es wurde eine Verdünnungsstufe pro Ansatz ausplattiert.. Die Anzahl der CFU/mL blieb über den Zeitraum von 6 h konstant. Nach 22 h war ein prozentueller Rückgang der Lebendzellzahl von lediglich 30,3 % zu verzeichnen.

**Beispiel 2: Vergleichsversuche zur Toxizität verschiedener flüssiger Kationenaustauscher gegenüber biotechnologisch relevanten Mikroorganismen**

Dieses Beispiel zeigt die niedrigere Toxizität von unverzweigten Fettsäuren gegenüber anderen flüssigen Kationenaustauschern wie DEHPA sowie verzweigten und unverzweigten gesättigten Fettsäuren.

Zunächst wurde eine Vorkultur umfassend 20 ml LB-Medium in einem 100 ml-Schikanekolben mit einer Kryokultur des entsprechenden Stammes angeimpft. Die Kultur wurde über Nacht bei 37°C und Schütteln bei 200 Upm kultiviert und am nächsten Tag verwendet, um eine gleiche Hauptkultur auf eine OD von 0,2 anzuimpfen. Die Hauptkulturen (je 30mL LB-Medium) wurde anschließend unter den gleichen Bedingungen weiter inkubiert. Bei einer OD von 0,4 bis 0,5 wurde die Hauptkultur mit jeweils gleichen Volumina (30 ml) Lösungsmittel überschichtet und anschließend weiter inkubiert.

Für die Bestimmung der Zahl der cfu (colony-forming units oder koloniebildende Einheiten) wurden bei den folgenden Versuchen 0,1 ml-Proben entnommen und in steriler 0,9 % NaCl-Lösung verdünnt. Geeignete Verdünnungsstufen wurden auf LB-Agarplatten ausplattiert. Nach Inkubation bei 34 °C über Nacht wurden die gebildeten Kolonien ausgezählt und die cfus bestimmt.

Versuch 1: Vergleich der Toxizität zwischen DE2HPA und einer gesättigten Fettsäure als flüssiger Kationenaustauscher

Es wurden 50 % DEHPA bzw. Laurinsäure (15%), jeweils in LSME gelöst und äquimolar bzw. 25 mol% mit ALSME beladen, als flüssige Kationenaustauscher mit einem *E. coli* BL21 (DE3)-Stamm kontaktiert und der Einfluss dieser beiden Verbindungen auf die Fähigkeit des Stammes untersucht, Kolonien zu bilden, ausgedrückt in cfus. In Vorversuchen konnte gezeigt werden, dass Laurinsäuremethylester - der aufgrund fehlender Ladung nicht als flüssiger Kationenaustauscher fungieren kann - von den verwendeten Stämmen gut vertragen wird.

**Tabelle 1:**

| Versuch Nr. | Verwendeter *E. coli*-Stamm | Verwendeter flüssiger Kationenaustauscher | Zahl der cfus nach 22 bzw. 24 h relativ zu t = 0 h |
|---|---|---|---|
| 1a | *E. coli* BL21(DE3) | Keiner | 244 % |
| 1b | *E. coli* BL21(DE3) | DEHPA | 0 % |
| 1c | *E. coli* BL21(DE3) | Laurinsäure | 1,2 % |

Es zeigt sich, dass beide flüssige Kationenaustauscher die Zahl der cfus deutlich senken, bei Verwendung von Laurinsäure im Gegensatz zu DEHPA aber noch einige lebensfähige Zellen vorhanden sind und die gesättigte Fettsäure als flüssiger Kationenaustauscher daher vorzuziehen ist.

Versuch 2: Vergleich der Toxizität zwischen verzweigten gesättigten Fettsäuren und verschiedenen Mengen Ölsäure als flüssiger Kationenaustauscher

Hierbei wurden zwei verschiedene Konzentrationen an Ölsäure eingesetzt und das Volumen durch Zugabe der entsprechenden Menge LSME (Laurinsäuremethylester) angepasst.

**Tabelle 2:**

| Versuch Nr. | Verwendeter *E. coli*-Stamm | Verwendeter flüssiger Kationenaustauscher | Zahl der cfus nach 22 bzw. 24 h relativ zu t = 0 h |
|---|---|---|---|
| 2a | *E. coli* BL21(DE3) | Isononansäure | 0 |
| 2b | *E. coli* BL21(DE3) | 2-Ethylhexansäure | 0 |
| 2c | *E. coli* BL21(DE3) | LSME/25% Ölsäure | 11 % |
| 2d | *E. coli* BL21(DE3) | LSME/75% Ölsäure | 18 % |
| 2e | *E. coli* W3110 | Isononansäure | 0 |
| 2f | *E. coli* W3110 | 2-Ethylhexansäure | 0 |
| 2g | *E. coli* W3110 | LSME/25% Ölsäure | 29 % |
| 2h | *E. coli* W3110 | LSME/75% Ölsäure | 17 % |

Es zeigt sich, dass die Zahl der lebensfähigen Zellen bei der Verwendung der ungesättigten Fettsäure Ölsäure zusammen mit LSME konsistent deutlich höher liegt als bei der Verwendung von verzweigten gesättigten Fettsäuren.

Versuch 3: Vergleich der Toxizität zwischen unverzweigten gesättigten Fettsäuren und ungesättigten Fettsäuren als flüssiger Kationenaustauscher

Hier wurden verschiedene Mengen einer ungesättigten Fettsäure mit einer ungesättigten Fettsäure verglichen hinsichtlich ihrer Toxizität bei der Verwendung als flüssiger Kationenaustauscher verglichen. Aufgrund der geringeren Löslichkeit der gesättigten Fettsäure Laurinsäure wurde diese in geringerer Menge eingesetzt. Die Volumina der verschiedenen Kationenaustauscher wurden mit LSME angeglichen. Die Zahl der cfus wurde zu Beginn, nach 4,5 h und nach 24 h bestimmt.

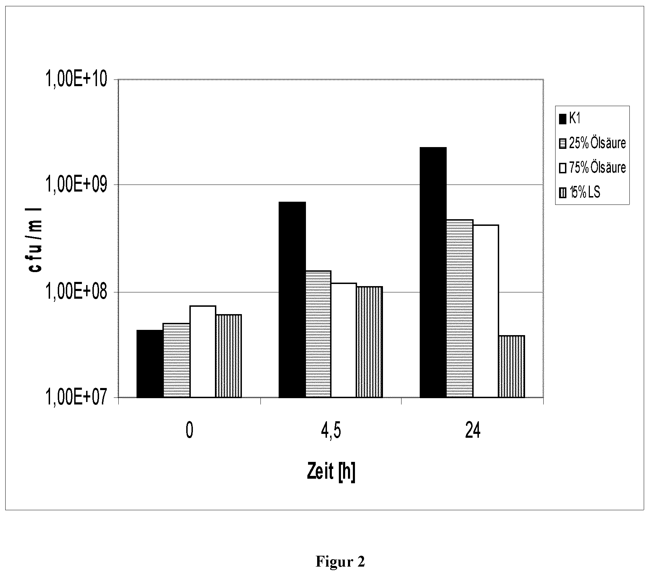

Wie aus Fig. 2 zu entnehmen ist, bewirkt die Zugabe der gesättigten Fettsäure als flüssigen Kationenaustauscher selbst in geringerer Konzentration als der der ungesättigten Fettsäure eine Abnahme der cfus, wohingegen im Falle der ungesättigten Fettsäure eine Zunahme der cfus festzustellen ist.

Es zeigt sich insgesamt eine Abnahme der Toxizität bei den verschiedenen untersuchten flüssigen Kationenaustauschern in folgender Reihenfolge: DEHPA > gesättigte Fettsäuren > ungesättigten Fettsäuren.

**Beispiel 3: Senkung der Toxizität einer organischen Verbindung mit positiver Ladung durch Kontaktieren mit einem flüssigen Kationenaustauscher**

Dieser Versuch zeigt, dass durch die Anwesenheit eines flüssigen Kationenaustauschers die toxische Wirkung einer positiv geladenen organischen Verbindung in einer wässrigen Phase, bei der es sich um Fermentationsbrühe handelt, gesenkt werden kann, indem diese Verbindung in die organische Phase extrahiert wird.

Das grundsätzliche experimentelle Vorgehen entsprach dem in Beispiel 1.

Da ALSME 0,2 % (w/v), in wässrigen Systemen gelöst, bakterizid wirkte, wurde dieser Versuch in Kombination mit D2EHPNH3/LSME 2/98 % (w/w) erneut im Schüttelkolben durchgeführt, dabei bedeutet D2EHPNH3 quantitativ mit Ammonium beladenes D2EHPA.. Durch den Einsatz des flüssigen Ionenaustauschers wird der Übergang von ALSME in die organische Phase verbessert, wodurch sich dessen Konzentration in der wässrigen Phase, in der sich auch die Zellen befinden, verringert. Um eine toxische Wirkung bedingt durch D2EHPA zu verringern, wurden niedrige Konzentrationen von 2 % (w/w) D2EHPNH3 verwendet.

Die Bakterien wurden zunächst in 5 mL, (entspricht der Hälfte des Puffervolumens) resuspendiert. Weitere 5 mL Puffer wurden ggf. mit 0,4 % (w/v) ALSME versetzt und anschließend ggf. mit 5 mL D2EHPNH3/LSME 2/98 % (w/w) 1 min bei 3000 Upm| gevortext. Diese Lösung wurde zu der im Schüttelkolben vorgelegten Bakteriensuspension hinzu gegeben und vermischt. Danach erfolgte die erste Probenahme.

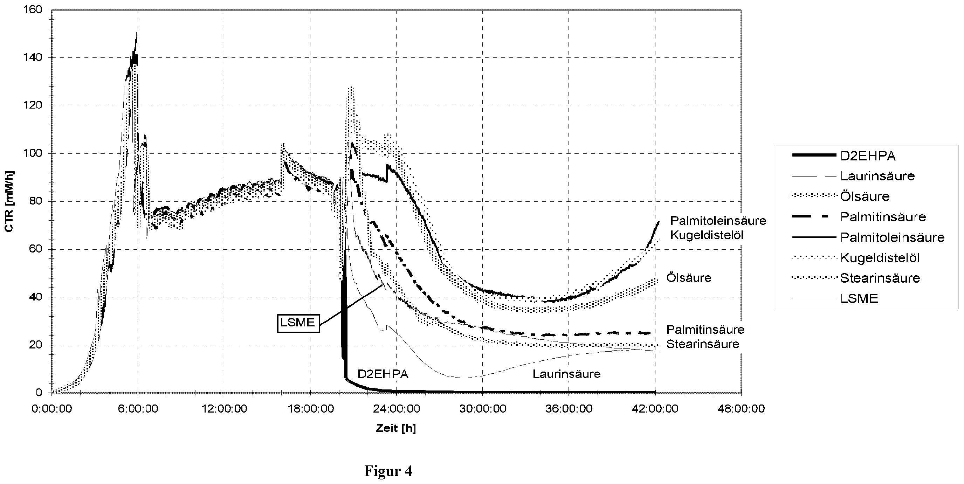

Die Lösung hatte eine schaumige Konsistenz zu Beginn der Versuche, die jedoch bei der 2. Probenahme in beiden Versuchen verschwunden war. Die Abkürzung "D/L" wurde für D2EHPNH3 (mit Ammoniak beladenes D2EHPA)/LSME 2/98 % (w/w) verwendet. Zwischen den Probenahmen $t_0$ und $t_{1,5}$ h erhöhte sich die Anzahl der CFU/mL um 34,3 %. Von der Probenahme ($t_{1,5}$) bis zur letzten Probenahme ($t_{22}$) verringerte sich die Anzahl der CFU/mL um 54,9 %. Im Vergleich zum Ansatz mit D2EHPNH3/LSME 2/98 % (w/w) ohne Zusatz von ALSME 0,2 % (w/v), war die Anzahl der vermehrungsfähigen Zellen nach 22 h 4,5 mal höher und mit 3,4 % nicht signifikant niedriger als der Mittelwert der Kontrollansätze in HEPES-Puffer (siehe Fig. 4). Im Vergleich zu dem Ansatz mit ALSME 0,2 % (w/v) im Schüttelkolben, ohne Hinzugabe einer organischen Phase, war die Anzahl der CFU/mL 2800-mal höher.

Es zeigt sich, dass die Anwesenheit des flüssigen Kationenaustauschers die Toxizität der positiv geladenen Verbindung, hier festgestellt durch die Zahl der verbliebenen cfus, verringert.

**Beispiel 4: Vergleichsversuche zur Toxizität verschiedener flüssiger Kationenaustauscher gegenüber einem ω-Aminolaurinsäure (ALS) und dem Methylester (ALSME) produzierenden Mikroorganismus**

Die Biotransformation von Laurinsäuremethylester zu Aminolaurinsäuremethylester wurde im 8-fach Paralellfermentationssystem von DasGip mit verschiedenen Ionentauschern getestet.

Für die Fermentation wurden 1L-Reaktoren verwendet. Die pH-Sonden wurden mittels einer Zwei-Punkt-Kalibration mit Maßlösungen von pH 4,0 und pH 7,0 kalibriert. Die Reaktoren wurden mit 300 mL Wasser befüllt und 20 min bei 121°C autoklaviert, um Sterilität zu gewährleisten. Anschließend wurden die pO2-Sonden über Nacht (mindestens 6 h lang) polarisiert. Am nächsten Morgen wurde das Wasser unter der Clean Bench entnommen und durch Hochzelldichtemedium mit 50 mg/L Kanamycin und 34 mg/L Chloramphenicol ersetzt. Im folgenden wurden die pO2-Sonden mit einer Einpunkt-Kalibration (Rührer: 600 rpm / Begasung: 10 sL/h Luft) kalibriert und die Feed-, Korrekturmittel- und Induktionsmittelstrecken mittels Clean-in-Place gereinigt. Dazu wurden die Schläuche mit 70% Ethanol, anschließend mit 1 M NaOH, dann mit sterilem VE-Wasser gespült und zuletzt mit den jeweiligen Medien befüllt.

Der ALS und ALSME produzierende *E. coli*-Stamm BL21 (DE3) T1r pBT10 pACYC:Duet[TAcv] wurde zuerst aus Cryokultur in LB-Medium (25 mL in einem 100 mL Schikanekolben) mit 50 mg/L Kanamycin und 34 mg/L Chloramphenicol über Nacht bei 37°C und 200 rpm ca. 18 h lang angezogen. Anschließend wurden je 2 mL in Hochzelldichtemedium (Glucose 15 g/L (30 mL / L einer separat autoklavierten 500 g/L Stammlösung mit 1% $MgSO_4*7H_2O$ und 2,2% $NH_4Cl$), $(NH_4)_2SO4$ 1,76 g/L, $K_2HPO_4$ 19,08 g/L, $KH_2PO_4$ 12,5 g/L, Hefeextrakt 6,66 g/L, Trinatriumcitrat-Dihydrat 11,2 g, Ammoniumeisencitrat-Lösung 17 mL/L einer separat autoklavierten 1% igen Stammlösung, Spurenelementlösung 5 mL/L separat autoklavierten Stammlösung (HCl (37 %) 36,50 g/L, $MnCl_2*4H_2O$ 1,91 g/L, $ZnSO_4*7H_2O$ 1,87 g/L, Ethylendiamintetraessigsäure-Dihydrat 0,84 g/L, $H_3BO_3$ 0,30 g/L. $Na_2MoO_4*2H_2O$ 0,25 g/L, $CaCl_2*2H_2O$ 4,70 g/L, $FeSO_4*7H_2O$ 17,80 g/L, $CuCl_2*2H_2O$ 0,15 g/L)) (3 mal je 25mL in einem 100 mL Schikanekolben) mit 50 mg/L Kanamycin und 34 mg/L Chloramphenicol überimpft und bei 37°C / 200 rpm weitere 6 h lang inkubiert.

Die 3 Kulturen wurden in einem Schüttelkolben vereinigt und die optische Dichte mit 7,2 bestimmt. Um die Reaktoren mit einer optischen Dichte von 0,1 anzuimpfen wurden je 4,2 mL in einer 5 mL Spritze aufgezogen und die Reaktoren mittels Kanüle über ein Septum beimpft.

Folgendes Standardprogramm wurde verwendet:

| DO-Regler | | pH-Regler | |
|-----------|------|-----------|---------|
| Preset | 0% | Preset | 0 ml/h |
| P | 0,1 | P | 5 |
| Ti | 300 s | Ti | 200 s |

(fortgesetzt)

| DO-Regler | | pH-Regler | |
|---|---|---|---|
| Preset | 0% | Preset | 0 ml/h |
| Min | 0% | Min | 0 mlL/h |
| Max | 100% | Max | 40 mL/h |

| N (Rotation) | von | bis | XO2 (Gasmischung) | von | bis | F (Gasfluss) | von | bis |
|---|---|---|---|---|---|---|---|---|
| | 0% | 30% | | 0% | 100% | | 15% | 80% |
| Wachstum und Biotransformation | 400 rpm | 1500 rpm | Wachstum und Biotransformation | 21% | 21% | Wachstum und Biotransformation | 6 sL/h | 72 sL/h |

| Script | |
|---|---|
| Trigger scharf | 31% DO (1/60h) |
| Induktion IPTG | 2 h nach Feedtstart |
| Feedtrigger | 50% DO |
| Feedrate | 3 [mL/h] |

Das durchgeführte Experiment lässt sich in zwei Phasen gliedern, die Anzucht, bei der die Zellen eine bestimmte optische Dichte erreichen sollten, und die nachfolgende Biotransformation, bei der die Expression der für das biotechnologische Verfahren zur Herstellung von ALSME erforderlichen Gene induziert wurde. Der pH Werte wurde einseitig mit Ammoniak (12,5 %) auf pH 6,8 geregelt. Während Anzucht und Biotransformation wurde der gelöste Sauerstoff (DO, dissolved oxygen) in der Kultur über Rührerdrehzahl und Begasungsrate bei 30% geregelt. Die Fermentation wurde als Feedbatch durchgeführt, wobei der Feedstart, 5 g/Lh Glucosefeed (500 g/L Glucose mit 1% $MgSO_4*7H_2O$ und 2,2% $NH_4Cl$), über einen DO-Peak getriggert wurde. Mit Feedstart wurde auch die Temperatur von 37°C auf 30°C gesenkt. Die Expression der Transaminase wurde 2 h nach Feedstart durch die automatische Zugabe von IPTG (1 mM) induziert. Die Induktion der alk-Gene erfolgte durch die manuelle Zugabe von DCPK (0,025 % v/v) 10 h nach Feedstart. Vor Start der Biotrasformation wurde die optische Dichte der Kulturbrühen bestimmt.

Der Start der Biotransformationsphase erfolgte 14 h nach Feedstart. Dazu wurden 150 mL eines Gemischs aus Laurinsäuremethylesther und dem jeweiligen Ionentauscher (10% w/w) als Batch zu der Fermentationsbrühe zugegeben. Als Ionenaustauscher wurden Di-(2-ethylhexyl-)phosphorsäure (DEHPA), Laurinsäure, Ölsäure, Palmitinsäure, Palmitoleinsäure, Stearinsäure und ein Gemisch aus freien Fettsäuren aus der Verseifung von Kugeldistelöl eingesetzt. Um einen Aminogruppendonor für die Transaminase zur Verfügung zu stellen wurden zeitgleich mit der Zugabe der organischen Phase 10,7 mL einer Alaninlösung (125 g/L) zu der Fermentationsbrühe zugegeben. Zur Probennahme wurden 2 mL Fermentationsbrühe aus dem Kessel entnommen und ein Teil davon 1/20 in einem Aceton-HCl-Gemisch (c(HCl) = 0,1 mol/L) verdünnt und extrahiert. Proben wurden bei 1,25 h, 3 h, 5 h, 20 h, 22 h und 25 h nach Start der Biotransformation von allen 8 Reaktoren genommen. Die Unsatzraten für Sauerstoff (OTR = oxygen transfer rate) und Kohlenstoff (CTR = carbon transfer rate) wurden währen der Fermentation über die Abgasanalytik an den DasGip-Systemen bestimmt. Die Fermentation wurde 22 h nach Start der Biotransformation beendet.

Die Quantifizierung von ALS, ALSME, DDS, DDSME, LS, LSME, HLS, HLSME, OLS und OLSME in Fermentationsproben erfolgte mittels LC-ESI/MS[2] anhand einer externen Kalibrierung für alle Analyten und unter Verwendung des internen Standards Aminoundekansäure (AUD).

Dabei kamen folgende Geräte zum Einsatz:

- HPLC Anlage 1260 (Agilent; Böblingen) mit Autosampler (G1367E), binärer Pumpe (G1312B) und Säulenofen (G1316A)
- Massenspektrometer TripelQuad 6410 (Agilent; Böblingen) mit ESI-Quelle
- HPLC-Säule: Kinetex C18, 100 x 2,1 mm, Partikelgröße: 2,6 $\mu$m, Porengröße 100 Å (Phenomenex; Aschaffenburg)
- Vorsäule: KrudKatcher Ultra HPLC In-Line Filter; 0,5 $\mu$m Filtertiefe und 0,004 mm Innendurchmesser (Phenomenex;

Aschaffenburg)

Die Proben wurden vorbereitet, indem 1900 μL Lösungsmittel (Aceton/ 0,1 N HCl-Gemisch = 1:1) und 100 μL Probe in ein 2-mL-Reaktionsgefäß pipettiert wurden. Das Gemisch wurde ca. 10 Sekunden gevortext und anschließend bei ca. 13000 rpm für 5 min zentrifugiert. Der klare Überstand wurde mit einer Pipette entnommen und nach entsprechender Verdünnung mit Diluent (80% (v/v) ACN, 20% bidest. $H_2O$ (v/v), + 0.1% Ameisensäure) analysiert. Zu je 900 μL Probe wurden 100 μL ISTD hinzupipettiert (10 μL bei einem Probenvolumen von 90 μL).

Die HPLC-Trennung erfolgte mit oben genannter Säule bzw. Vorsäule. Das Injektionsvolumen betrug 0,7 μL, die Säulentemperatur 50°C, die Flussrate 0,6 mL/min. Die mobile Phase bestand aus Eluent A (0,1%ige (v/v) wässrige Ameisensäure) und Eluent B (Acetonitril mit 0,1% (v/v) Ameisensäure). Folgendes Gradientenprofil wurde genutzt

| Zeit [min] | Eluent A [%] | Eluent B [%] |
|---|---|---|
| 0 | 77 | 23 |
| 0.3 | 77 | 23 |
| 0.4 | 40 | 60 |
| 2.5 | 40 | 60 |
| 2.6 | 2 | 98 |
| 5.5 | 2 | 98 |
| 5.6 | 77 | 23 |
| 9 | 77 | 23 |

Die ESI-MS[2]-Analyse erfolgte im positiven Modus mit folgenden Parametern der ESI-Quelle:

- Gastemperatur 280°C
- Gasfluss 11 L/min
- Nebulizerdruck 50 psi
- Kapillarspannung 4000 V

Die Detektion und Quantifizierung der einzelnen Verbindungen erfolgte mit den folgenden Parametern, wobei jeweils ein Produkt-Ion als *Qualifier* und eines als *Quantifier* genutzt wurde:

| Analyt | Vorläufer-Ion [m/z] | Produkt-Ion [m/z] | Verweilzeit [ms] | Kollisionsenergie [eV] |
|---|---|---|---|---|
| DDSME | 245,2 | 167,1 | 25 | 6 |
| DDSME | 245,2 | 149,1 | 50 | 8 |
| HLSME | 231,3 | 181,2 | 15 | 2 |
| HLSME | 231,3 | 163,2 | 25 | 5 |
| DDS | 231,2 | 213,2 | 50 | 0 |
| DDS | 231,2 | 149,1 | 25 | 9 |
| ALSME | 230,3 | 198,1 | 25 | 10 |
| ALSME | 230,3 | 163,2 | 15 | 10 |
| OLSME | 229,2 | 197,2 | 50 | 0 |
| OLSME | 229,2 | 161,1 | 25 | 5 |
| HLS | 217,2 | 181,2 | 35 | 0 |
| HLS | 217,2 | 163,1 | 20 | 4 |
| OLS | 215,2 | 161,2 | 25 | 0 |
| OLS | 215,2 | 95,2 | 60 | 13 |

Ergebnisse:

Wird DEHPA wie im Stand der Technik beschrieben als Kationenaustauscher eingesetzt, so kommt es unmittelbar nach Zusatz der Verbindung zur Kultur zu einem Einbruch der OTR. Die Kurve sinkt innerhalb kurzer Zeit auf 0, was anzeigt, dass in der Kultur keine stoffwechselaktiven Zellen mehr vorhanden sind. DEHPA wirkt also hochgradig toxisch auf Zellen.

Wird statt DEHPA Laurinsäure als flüssiger Kationenaustauscher eingesetzt, so kommt es zwar ebenfalls zu einem Einbruch der OTR, dieser ist jedoch nicht so stark, und im Verlauf der nächsten 22 h erholen sich die Zellen und zeigen eine steigende Stoffwechselaktivität. Laurinsäure ist demnach merklich weniger toxisch als DEHPA.

Noch deutlich bessere Ergebnisse können bei Verwendung gesättigter Fettsäuren mit längeren Kohlenstoffketten beobachtet werden. Werden Palmitin- und Stearinsäure eingesetzt, so fällt die OTR-Kurve deutlich seichter als im Falle der Verwendung von Laurinsäure oder gar DEHPA. Daraus kann geschlossen werden, dass diese Fettsäuren deutlich weniger toxisch wirken.

Die Verwendung von ungesättigten Fettsäuren wie Palmitoleinsäure, verseiftem Kugeldistelöl (enthält überwiegend Linolsäure) und Ölsäure führt überraschend zu noch deutlich besseren Ergebnissen. Diese Fettsäuren zeigen überraschend eine noch geringere Toxizität als die gesättigten Fettsäuren.

**Literaturstellen:**

J. Sangster, Octanol-Water Partition Coefficients: Fundamentals and Physical Chemistry, Vol. 2 of Wiley Series in Solution Chemistry, John Wiley & Sons, Chichester, 1997

Asano, Y., Fukuta, y., Yoshida, Y., and Komeda, H. (2008): The Screening, Characterisation, and Use of w-Laurolactam Hydrolase: A New Enzymatic Synthesis of 12-Aminolauric Acid, Biosc. Biotechn. Biochem., 72 (8), 2141-2150

DE10200710060705 (2007): $\omega$-Aminocarbonsäuren oder ihre Lactame, herstellende, rekombinante Zellen

F. M. Ausubel (1995), Current Protocols in Molecular Biology. John Wiley & Sons, Inc.

A. M. Lesk (2008), Introduction to Bioinformatics, 3rd Edition

**Patentansprüche**

1. Verfahren zum Entfernen einer organischen Verbindung aus einer wässrigen Lösung, umfassend die Schritte

   a) Bereitstellen der die organische Verbindung enthaltenden wässrigen Lösung und einer hydrophoben organischen Lösung, die einen flüssigen Kationenaustauscher umfasst, wobei der flüssige Kationenaustauscher hydrophob ist,
   b) Kontaktieren der wässrigen Lösung und der organischen Lösung, und
   c) Abtrennen der organischen Lösung von der wässrigen Lösung,

   wobei es sich bei der organischen Verbindung um eine Verbindung der Formel I

$$NH_3{}^+\text{-}A\text{-}COOR^1 \qquad (I),$$

   handelt, wobei $R^1$ Wasserstoff, Methyl, Ethyl oder eine negative Ladung ist und A eine unsubstituierte, geradekettige Alkylengruppe mit wenigstens drei, bevorzugt wenigstens acht Kohlenstoffatomen ist, und wobei es sich bei dem flüssigen Kationenaustauscher um eine ungesättigte Fettsäure handelt.

2. Verfahren nach einem der Ansprüche 1, wobei die Temperatur bei Schritt b) 28 bis 70, bevorzugt 30 bis 37 °C beträgt.

3. Verfahren nach einem der Ansprüche 1 bis 2, wobei der pH-Wert bei Schritt b) 6 bis 8, bevorzugt 6,2 bis 7,2, beträgt.

4. Verfahren nach einem der Ansprüche 1 bis 3, wobei das Stoffmengenverhältnis von flüssigem Kationenaustauscher zu organischer Verbindung wenigstens 1 beträgt.

5. Verfahren nach einem der Ansprüche 1 bis 4, wobei das Volumenverhältnis von organischer Lösung zu wässriger Lösung 1:10 bis 10:1 beträgt.

6. Verfahren nach einem der Ansprüche 1 bis 5, wobei der flüssige Kationenaustauscher eine Fettsäure mit mehr als 12, bevorzugt mit 14 bis 22, noch bevorzugter 16 bis 18 Kohlenstoffatomen ist.

**7.** Verfahren nach einem der Ansprüche 1 bis 6, wobei der flüssige Kationenaustauscher Ölsäure oder Erukasäure ist.

**8.** Verfahren nach einem der Ansprüche 1 bis 7, wobei die wässrige Lösung weiterhin eine Zelle umfasst.

**9.** Verfahren nach Anspruch 8, wobei die Zelle eine bakterielle Zelle ist und die Zelle noch bevorzugter eine rekombinante Alkanmonooxygenase, eine rekombinante Transaminase sowie bevorzugt darüber hinaus wenigstens ein Enzym aus der Gruppe aufweist, die eine Alkoholdehydrogenase, eine Alanindehydrogenase und das AlkL-Genprodukt umfasst.

**10.** Verfahren nach einem der Ansprüche 1 bis 9, wobei die organische Lösung darüber hinaus mindestens ein organisches Lösungsmittel enthält, bevorzugt eine Fettsäure und/oder einen Fettsäureester.

**11.** Verfahren nach Anspruch 10, wobei die organische Lösung als flüssigen Kationenaustauscher 20 bis 80 Vol.-%, bevorzugt 25 bis 75 Vol.-% Ölsäure, und als Lösungsmittel Laurinsäuremethylester umfasst und es sich bei der organischen Verbindung um 12-Aminolaurinsäuremethylester handelt und in der wässrigen Lösung eine bakterielle Zelle anwesend ist, die eine rekombinante Alkanmonooxygenase, eine rekombinante Transaminase sowie bevorzugt darüber hinaus wenigstens ein Enzym aus der Gruppe aufweist, die eine Alkoholdehydrogenase, eine Alanindehydrogenase und das AlkL-Genprodukt umfasst.

**12.** Reaktionsmischung umfassend eine wässrige Lösung und eine hydrophobe organische Lösung, wobei die hydrophobe organische Lösung eine ungesättigte Fettsäure, bevorzugter eine Fettsäure mit mehr als 12 Kohlenstoffatomen, als flüssigen Kationenaustauscher umfasst, und wobei die wässrige Lösung eine Verbindung der Formel (I)

$$NH_3{}^+\text{-}A\text{-}COOR^1 \qquad (I),$$

enthält, wobei $R^1$ Wasserstoff, Methyl, Ethyl oder eine negative Ladung ist und A eine unsubstituierte, geradekettige Alkylengruppe mit wenigstens drei, bevorzugt wenigstens acht Kohlenstoffatomen ist.

**13.** Reaktionsmischung nach Anspruch 12, wobei die wässrige Lösung weiterhin eine Zelle umfasst, die eine rekombinante Alkanmonooxygenase, eine rekombinante Transaminase sowie bevorzugt darüber hinaus wenigstens ein Enzym aus der Gruppe aufweist, die eine Alkoholdehydrogenase, eine Alanindehydrogenase und das AlkL-Genprodukt umfasst.

**Claims**

**1.** Method for removing an organic compound from an aqueous solution, comprising the steps

 a) providing the aqueous solution which contains the organic compound, and a hydrophobic organic solution which comprises a liquid cation exchanger,
 wherein the liquid cation exchanger is hydrophobic,
 b) contacting the aqueous solution and the organic solution, and
 c) separating off the organic solution from the aqueous solution,

wherein the organic compound is a compound of the formula I

$$NH_3{}^+\text{-}A\text{-}COOR^1 \qquad (I)$$

wherein $R^1$ is hydrogen, methyl, ethyl or a negative charge and A is an unsubstituted, straight-chain alkylene group having at least three, preferably at least eight, carbon atoms,
and wherein the liquid cation exchanger is an unsaturated fatty acid.

**2.** Method according to any one of Claims 1, wherein the temperature in step b) is 28 to 70, preferably 30 to 37°C.

**3.** Method according to either of Claims 1 to 2, wherein the pH in step b) is 6 to 8, preferably 6.2 to 7.2.

**4.** Method according to any one of Claims 1 to 3, wherein the molar ratio of liquid cation exchanger to organic compound is at least 1.

**5.** Method according to any one of Claims 1 to 4, wherein the volumetric ratio of organic solution to aqueous solution is 1:10 to 10:1.

**6.** Method according to any one of Claims 1 to 5, wherein the liquid cation exchanger is a fatty acid having more than 12, preferably having 14 to 22 and even more preferably 16 to 18 carbon atoms.

**7.** Method according to any one of Claims 1 to 6, wherein the liquid cation exchanger is oleic acid or erucic acid.

**8.** Method according to any one of Claims 1 to 7, wherein the aqueous solution additionally comprises a cell.

**9.** Method according to Claim 8, wherein the cell is a bacterial cell and the cell more preferably includes a recombinant alkane monooxygenase, a recombinant transaminase and preferably additionally at least one enzyme from the group comprising an alcohol dehydrogenase, an alanine dehydrogenase and the AlkL gene product.

**10.** Method according to any one of Claims 1 to 9, wherein the organic solution additionally contains at least one organic solvent, preferably a fatty acid and/or a fatty acid ester.

**11.** Method according to Claim 10, wherein the organic solution comprises, as liquid cation exchanger, 20 to 80% by volume, preferably 25 to 75% by volume, of oleic acid, and, as solvent, methyl laurate, and the organic compound is methyl 12-aminolaurate and a bacterial cell is present in the aqueous solution, including a recombinant alkane monooxygenase, a recombinant transaminase and preferably additionally at least one enzyme from the group comprising an alcohol dehydrogenase, an alanine dehydrogenase and the AlkL gene product.

**12.** Reaction mixture comprising an aqueous solution and a hydrophobic organic solution,
wherein the hydrophobic organic solution comprises an unsaturated fatty acid, more preferably a fatty acid having more than 12 carbon atoms, as liquid cation exchanger,
and wherein the aqueous solution contains a compound of the formula (I)

$$NH_3^+\text{-A-COOR}^1 \qquad (I),$$

wherein $R^1$ is hydrogen, methyl, ethyl or a negative charge and A is an unsubstituted, straight-chain alkylene group having at least three, preferably at least eight, carbon atoms.

**13.** Reaction mixture according to Claim 12, wherein the aqueous solution additionally comprises a cell including a recombinant alkane monooxygenase, a recombinant transaminase and preferably additionally at least one enzyme from the group comprising an alcohol dehydrogenase, an alanine dehydrogenase and the AlkL gene product.

**Revendications**

**1.** Procédé d'élimination d'un composé organique d'une solution aqueuse, comprenant les étapes suivantes :

a) la préparation de la solution aqueuse contenant le composé organique et d'une solution organique hydrophobe qui comprend un échangeur de cations liquide, l'échangeur de cations liquide étant hydrophobe,
b) la mise en contact de la solution aqueuse et de la solution organique, et
c) la séparation de la solution organique de la solution aqueuse,

le composé organique étant un composé de formule I

$$NH_3^+\text{-A-COOR}^1 \qquad (I)$$

dans laquelle $R^1$ représente hydrogène, méthyle, éthyle ou une charge négative, et A représente un groupe alkylène linéaire non substitué contenant au moins trois, de préférence au moins huit atomes de carbone, et l'échangeur de cations liquide étant un acide gras insaturé.

**2.** Procédé selon l'une quelconque des revendications 1, dans lequel la température à l'étape b) est de 28 à 70, de préférence de 30 à 37 °C.

**3.** Procédé selon l'une quelconque des revendications 1 à 2, dans lequel le pH à l'étape b) est de 6 à 8, de préférence de 6,2 à 7,2.

**4.** Procédé selon l'une quelconque des revendications 1 à 3, dans lequel le rapport entre les quantités de matière d'échangeur de cations liquide et de composé organique est d'au moins 1.

**5.** Procédé selon l'une quelconque des revendications 1 à 4, dans lequel le rapport volumique entre la solution organique et la solution aqueuse est de 1:10 à 10:1.

**6.** Procédé selon l'une quelconque des revendications 1 à 5, dans lequel l'échangeur de cations liquide est un acide gras contenant plus de 12, de préférence 14 à 22, de manière encore davantage préférée 16 à 18 atomes de carbone.

**7.** Procédé selon l'une quelconque des revendications 1 à 6, dans lequel l'échangeur de cations liquide est l'acide oléique ou l'acide érucique.

**8.** Procédé selon l'une quelconque des revendications 1 à 7, dans lequel la solution aqueuse comprend en outre une cellule.

**9.** Procédé selon la revendication 8, dans lequel la cellule est une cellule bactérienne et la cellule comprend de manière encore davantage préférée une alcane-monooxygénase recombinante, une transaminase recombinante, et de préférence en outre au moins une enzyme du groupe comprenant une alcool-déshydrogénase, une alanine-déshydrogénase et le produit génique AlkL.

**10.** Procédé selon l'une quelconque des revendications 1 à 9, dans lequel la solution organique contient en outre au moins un solvant organique, de préférence un acide gras et/ou un ester d'acide gras.

**11.** Procédé selon la revendication 10, dans lequel la solution organique comprend en tant qu'échangeur de cations liquide 20 à 80 % en volume, de préférence 25 à 75 % en volume, d'acide oléique, et en tant que solvant de l'ester méthylique de l'acide laurique, et le composé organique est l'ester méthylique de l'acide 12-aminolaurique, et une cellule bactérienne est présente dans la solution aqueuse, qui comprend une alcane-monooxygénase, une transaminase recombinante, et de préférence en outre au moins une enzyme du groupe comprenant une alcool-déshydrogénase, une alanine-déshydrogénase et le produit génique AlkL.

**12.** Mélange réactionnel comprenant une solution aqueuse et une solution organique hydrophobe, la solution organique hydrophobe comprenant un acide gras insaturé, de préférence un acide gras contenant plus de 12 atomes de carbone, en tant qu'échangeur de cations liquide, et la solution aqueuse comprenant un composé de formule (I)

$$NH_3{}^+\text{-A-COOR}^1 \qquad (I)$$

dans laquelle $R^1$ représente hydrogène, méthyle, éthyle ou une charge négative, et A représente un groupe alkylène linéaire non substitué contenant au moins trois, de préférence au moins huit atomes de carbone.

**13.** Mélange réactionnel selon la revendication 12, dans lequel la solution aqueuse comprend en outre une cellule, qui comprend une alcane-monooxygénase recombinante, une transaminase recombinante, et de préférence en outre au moins une enzyme du groupe comprenant une alcool-déshydrogénase, une alanine-déshydrogénase et le produit génique AlkL.

**Figur 1**

**Figur 2**

Figur 3

Figur 4

**Figur 5**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10200710060705
- WO 9802411 A
- EP 11004029 A
- EP 2011053834 W

**In der Beschreibung aufgeführte Nicht-Patentliteratur**

- Octanol-Water Partition Coefficients: Fundamentals and Physical Chemistry. **J. SANGSTER.** Wiley Series in Solution Chemistry. John Wiley & Sons, 1997, vol. 2
- **ASANO, Y. ; FUKUTA, Y. ; YOSHIDA, Y. ; KOMEDA, H.** The Screening, Characterisation, and Use of w-Laurolactam Hydrolase: A New Enzymatic Synthesis of 12-Aminolauric Acid. *Biosc. Biotechn. Biochem.,* 2008, vol. 72 (8), 2141-2150
- **F. M. AUSUBEL.** Current Protocols in Molecular Biology. John Wiley & Sons, Inc, 1995
- **A. M. LESK.** Introduction to Bioinformatics. 2008